(19) Europäisches Patentamt
European Patent Office
Office européen des brevets

(11) **EP 2 133 048 B1**

(12) **EUROPÄISCHE PATENTSCHRIFT**

(45) Veröffentlichungstag und Bekanntmachung des Hinweises auf die Patenterteilung:
**13.03.2019 Patentblatt 2019/11**

(51) Int Cl.:
***A61F 9/013*** *(2006.01)*

(21) Anmeldenummer: **09012579.0**

(22) Anmeldetag: **14.03.2007**

(54) **Apparat zur Ankopplung eines Elementes an das Auge**

Apparatus for connecting an element to an eye

Appareil destiné au couplage d'un élément sur l'oeil

(84) Benannte Vertragsstaaten:
**DE ES FR GB IT**

(43) Veröffentlichungstag der Anmeldung:
**16.12.2009 Patentblatt 2009/51**

(62) Dokumentnummer(n) der früheren Anmeldung(en) nach Art. 76 EPÜ:
**07005280.8 / 1 970 034**

(73) Patentinhaber: **WaveLight GmbH**
**91058 Erlangen (DE)**

(72) Erfinder:
• **Donitzky, Christof**
**90542 Eckental (DE)**
• **Wüllner, Christian**
**91094 Bräuningshof (DE)**

(74) Vertreter: **Katérle, Axel**
**Wuesthoff & Wuesthoff**
**Patentanwälte PartG mbB**
**Schweigerstraße 2**
**81541 München (DE)**

(56) Entgegenhaltungen:
| | |
|---|---|
| **EP-A1- 0 327 693** | **WO-A-2004/096106** |
| **WO-A-2005/048895** | **WO-A-2006/090217** |
| **WO-A-2006/121066** | **WO-A2-93/14817** |
| **US-A- 4 546 773** | **US-A- 6 126 668** |
| **US-A1- 2005 192 562** | |

EP 2 133 048 B1

**Beschreibung**

Die Erfindung betrifft eine Vorrichtung für die Augenchirurgie, wobei die Vorrichtung einen auf ein zu behandelndes Auge aufsetzbaren Saugring mit einem Saugbereich umfasst, der dazu ausgebildet ist, durch Unterdruckerzeugung in dem Saugbereich den Saugring an das Auge zu saugen.

Gepulste Laserstrahlung wird in der Augenchirurgie beispielsweise zum Anbringen von Schnitten in der Hornhaut (Kornea) oder zum Abtrag (Ablation) von Gewebe aus der Hornhaut verwendet. Die eingestrahlte Laserstrahlung bewirkt im Hornhautgewebe einen photodisruptiven bzw. photoablativen Prozess, der zur Gewebetrennung bzw. zur Entfernung von Gewebematerial führt. Derartige Bearbeitungen der Kornea finden beispielsweise im Rahmen von refraktiven Verfahren zur Minderung oder vollständigen Behebung von Fehlsichtigkeiten des Auges statt, bei denen die Kornea neu geformt wird und hierdurch ihre Brechungseigenschaften verändert werden.

Das dominierende refraktive Verfahren der Hornhautchirurgie ist die sogenannte LASIK (Laser in sito Keratomileusis). Hierbei wird aus der Hornhaut entweder mechanisch (mittels einer oszillierenden Schneidklinge in einem sogenannten Mikrokeratom) oder optisch (mittels Laserstrahlung, z.B. mittels Femtosekunden-Lasersysteme) ein kleiner Deckel herausgeschnitten, der mit einem Teil seines Randes noch an der Hornhaut hängt. Anschließend wird dieser üblicherweise auch als Flap bezeichnete Deckel zur Seite geklappt, wodurch das darunter liegende Stroma zugänglich wird. Mit Laserstrahlung wird dann nach Maßgabe eines für den jeweiligen Patienten ermittelten Ablationsprofils Stromagewebe abgetragen. Der Deckel wird danach wieder zurückgeklappt, wodurch die Wunde relativ schnell verheilen kann.

Für eine präzise Einkopplung der Laserstrahlung in das Auge ist hierbei bekannt, das Auge mittels einer Fixationsvorrichtung zu fixieren, welche durch Unterdruck am Auge angesaugt wird. Die Fixationsvorrichtung kann ein als Einkoppelelement für die Laserstrahlung dienendes Glas aufweisen. Derartige Fixationsvorrichtungen werden auch als Saugringe bezeichnet.

Sobald der Saugring am Auge eines Patienten angeordnet und mittels eines Unterdruckes an dem Auge fixiert ist, wird mittels Impulsen eines Femtosekunden-Lasers Energie in das Hornhautinnere eingebracht. Dadurch wird in der Hornhaut ein Schnitt erzeugt, der Flap kann aufgeklappt werden und die Korrektur der Fehlsichtigkeit kann durch einen definierten Abtrag von freigelegtem Hornhautgewebe erfolgen.

Saugringe per se sind dem Fachmann bekannt, beispielsweise offenbaren die US 5 336 215 und US 5 549 632 Saugringe, die in ihrem Umfangsbereich als Saugbereiche ausgebildete Öffnungen zum Ansaugen an ein Auge umfassen. Die EP 0 993 814 A1 sowie die US 6 342 053 B1 offenbaren Saugringe, bei denen ein Unterdruck im Bereich einer Applanationsfläche erzeugt wird, so dass die Hornhaut des Auges daran anliegt. Die US 6 344 040 B1 zeigt einen Saugring, bei dem ein Unterdruck im Bereich einer Applanationsfläche erzeugt wird, wobei der Saugring ferner eine Sonde umfasst, die im Einsatz die Hornhaut durchsticht und mittels einer Absaugung die während des photodisruptiven Verfahrens entstehenden Gase und Partikel absaugt. Die WO 03/002008 A1 offenbart einen Saugring mit einem am Umfang des Saugringes ausgebildeten Ansaugbereich, wobei an dem Saugring mittels eines zangenartigen Greifers eine konusförmige Linsenhalterung mit einer Linse angeordnet wird.

Die WO 00/41660 A1 beschreibt eine Einrichtung zum Durchführen einer Operation am Auge mit einem ersten ringförmigen, festen Vakuumbereich und einem zentralen, beweglichen Vakuumbereich. Der bewegliche Vakuumbereich befindet sich im Einsatz über der zu operierenden Hornhaut und kann dieser eine für die Operation gewünschte Form geben. Der zweite Vakuumbereich kann eine Mehrzahl von Elementen umfassen, so dass dessen Form und folglich die Kontur der Hornhaut während der Operation verändert werden kann.

Die WO 03/001991 A1 offenbart eine Kontaktlinse mit einer Mehrzahl von Dehnungsmesseinrichtungen zum Messen des Augeninnendruckes. Die Stromversorgung der Dehnungsmesseinrichtungen und die Kommunikation erfolgen berührungslos.

Die Saugringe des Standes der Technik umfassen Applanationselemente, die mittels aufwändiger mechanischer Vorrichtungen mit dem Saugring gekoppelt werden.

Der bei der Ansaugung des Saugringes wirkende Augeninnendruck kann zu Schäden führen und ist bisher dem Operateur während der Behandlung eines Auges nicht bekannt. Ferner ist die Positionierung des Saugringes, beispielsweise bezüglich eines Femtosekunden-Lasersystems kritisch, und kann unter Umständen durch Verfahren der Behandlungsliege zu Behandlungsfehlern oder Verletzungen führen. Die Position des Saugringes und des Auges, insbesondere bezüglich einer Behandlungsliege und des Laserstrahles, ist sicherheitsrelevant. Eine mechanische Kraft, die während der Laserbehandlung auf das Auge wirkt, wird unter Umständen durch die bei einer Kopfbewegung entstehenden mechanischen Kräfte beeinflusst und kann zu einer Verletzung führen. Bei der Behandlung können der Wassergehalt und/oder die biomechanischen Eigenschaften der Hornhaut wichtig für das Behandlungsergebnis sein. Die Transparenz der Hornhaut ist für die Bearbeitung mit Laserimpulsen ebenfalls wichtig, um die Operationssicherheit zu verbessern.

Aus der WO 2006/121066 A1 ist ein Aufsatzelement bekannt, das für eine Laserbehandlung eines Auges auf dieses aufgesetzt wird. In dem Aufsatzelement befindet sich im Abstand von der Augenvorderfläche ein Linsenelement mit Fokussierfunktion für die verwendete Laserstrahlung. Zwischen der Augenvorderfläche, dem Linsenelement und der Umfangswand des Aufsatzelements ist ein Kühlraum begrenzt, der mit einer Kühlflüssigkeit befüllt wird, um während der Laserbestrahlung des Auges den Augapfel zu kühlen. Zwei Löcher sind in der Umfangswand des Aufsatzelements vorgesehen, eines zum Einfüllen der Kühlflüssigkeit in den Kühlraum, das andere zum Auslassen der Kühlflüssigkeit. Die beiden Löcher sind in den Zeichnungen dieses Dokuments in gleicher axialer Höhe - bezogen auf eine gedachte Mittenachse des Aufsatzelements - gezeigt.

Die Erfindung stellt demgegenüber eine Vorrichtung für die Augenchirurgie bereit, die dazu ausgebildet ist, während einer Behandlung eines Auges ein von Luft verschiedenes Fluid zwischen einer Fokussierlinse und der Hornhaut des Auges bereitzuhalten, wobei die Vorrichtung umfasst: einen auf ein zu behandelndes Auge aufsetzbaren Saugring mit einem ersten Saugbereich, der dazu ausgebildet ist, durch Unterdruckerzeugung in dem ersten Saugbereich den Saugring an das Auge zu saugen, und ein integral mit dem Saugring ausgebildetes oder mittels Unterdruck oder mechanisch an den Saugring ankoppelbares Gefäßelement mit einer Längsachse und einem Innenraum zur Aufnahme des Fluids, wobei das Gefäßelement eine sich von einem ersten axialen Ende zu einem entgegengesetzten zweiten axialen Ende des Gefäßelements erstreckende, um die Längsachse verlaufende Wand aufweist, durch welche es seitlich geschlossen ist, und wobei in der Wand eine erste Öffnung zur Zuführung des Fluids in den Innenraum und eine zweite Öffnung zur Entlüftung des Innenraums vorgesehen sind, wobei die beiden Öffnungen axial zueinander versetzt in der Wand angeordnet sind.

Der Saugbereich kann beispielsweise durch eine Öffnung oder eine Ausnehmung gebildet sein, in der im Einsatz ein Unterdruck herrscht. Der Unterdruck kann beispielsweise durch eine mit dem Saugbereich verbundene Saugpumpe erzeugt werden.

Soweit das Gefäßelement mittels Unterdruck an den Saugring ankoppelbar ist, wird das erste axiale Ende des Gefäßelements im Einsatz durch Unterdruckerzeugung in einem zweiten Saugbereich des Saugrings an diesen angesaugt. Das Gefäßelement kann an seinem ersten axialen und zweiten axialen Ende geöffnet sein. Aufgrund des Gefäßelementes hat die Fokussierlinse einen festen Abstand von der Hornhaut, wodurch eine sichere Behandlung gewährleistet ist.

Wird durch die erste Öffnung ein Fluid eingeleitet, befindet sich in der Umgebung des ersten Saugbereiches ein Feuchtigkeitsfilm, der eine besonders gute Abdichtung des ersten Saugbereiches gewährleistet, wodurch die Augensaugeinrichtung besonders sicher an das Auge gekoppelt wird. Je höher die Viskosität der Flüssigkeit ist, desto besser ist die zusätzliche Dichtwirkung.

Das Gefäßelement kann mit einem Fluid gefüllt werden, dessen Brechungsindex etwa dem Brechungsindex der Hornhaut entspricht. Dadurch entsteht keine optische Aberration des Lichtes beim Übergang in die Hornhaut, wodurch eine gute Fokussierbarkeit des Laserstrahles und ein hohe optische Qualität des Laserstrahles gewährleistet ist. Das Fluid kann einen Brechungsindex $\eta_{Fluid}$ von etwa 1,35 bis etwa 1,40, vorzugsweise von etwa 1,36 bis 1,38, höchst vorzugsweise von etwa 1,37 aufweisen. Der Brechungsindex $\eta_{Cornea}$ der Hornhaut beträgt etwa 1,376, und falls der Brechungsindex des Fluids einen ähnlichen Brechungsindex aufweist, wird die Qualität und/oder die Intensität eines Lichtstrahles bzw. eines Laserstrahles beim Übergang vom optischen Augenkontaktelement in die Hornhaut nicht verringert.

Die Reflektionsverluste R werden wie folgt berechnet:

$$R = \left[ \frac{\eta_{Cornea} - \eta_{Fluid}}{\eta_{Cornea} + \eta_{Fluid}} \right]^2 ;$$

Bei $\eta_{Cornea} \approx \eta_{Fluid}$ ergibt sich, dass nahezu keine Reflektionsverluste auftreten.

Über die zweite Öffnung kann beim Befüllen des Gefäßelementes Luft austreten.

Ferner ist bei dieser Ausführungsform kein Applanationselement erforderlich, wodurch der Augendruck während der Behandlung nicht erhöht wird. Ferner kann keine Aberration, beispielsweise ein Wellenfrontfehler, aufgrund einer sphärischen oder asphärischen Applanation wie beim Einsatz eines Applanationselementes entstehen. Dadurch wird eine Laserstrahlung mit einem niedrigen Wellenfrontfehler erreicht, die hinsichtlich der Fokussierung vorteilhaft ist, da die Fokuspositionen weniger stark streuen und die Laserstrahlung auf einen kleineren Fokusbereich konzentriert wird.

Die Erfindung wird nachfolgend anhand der beigefügten Zeichnungen näher erläutert. Es stellen dar:

Figur 1 einen schematischen, nicht maßstabsgetreuen Schnitt durch ein erstes Beispiel einer augenchirurgischen Vorrichtung;

Figur 2a einen schematischen, nicht maßstabsgetreuen Schnitt durch ein zweites Beispiel einer augenchirurgischen Vorrichtung;

Figur 2b einen schematischen, nicht maßstabsgetreuen Schnitt durch ein Beispiel einer augenchirurgischen Vorrichtung, bei der sich ein zweiter Saugbereich an einem Funktionselement befindet;

Figur 3 eine schematische Frontansicht einer beispielhaften augenchirurgischen Vorrichtung;

Figur 4 eine schematische, nicht maßstabsgetreue Ansicht einer Ausführungsform der Erfindung, bei der sich während der Behandlung zwischen einer Fokussierlinse und der Hornhaut ein Fluid befindet;

Figur 5 eine schematische, nicht maßstabsgetreue Ansicht eines weiteren Beispiels einer augenchirurgischen Vorrichtungbei der ein Applanationselement an einem Saugring befestigt ist;

Figur 6 eine schematische, nicht maßstabsgetreue Ansicht eines weiteren Beispiels einer augenchirurgischen Vorrichtung, bei der ein Applanationselement an einem Saugring befestigt ist;

Figur 7 eine perspektivische, nicht maßstabsgetreue Ansicht eines weiteren Beispiels einer augenchirurgischen Vorrichtung, die einen Saugring mit einer Messeinrichtung umfasst; und

Figur 8 einen schematischen, nicht maßstabsgetreuen Schnitt durch die Beispielvorrichtung gemäß Fig. 7.

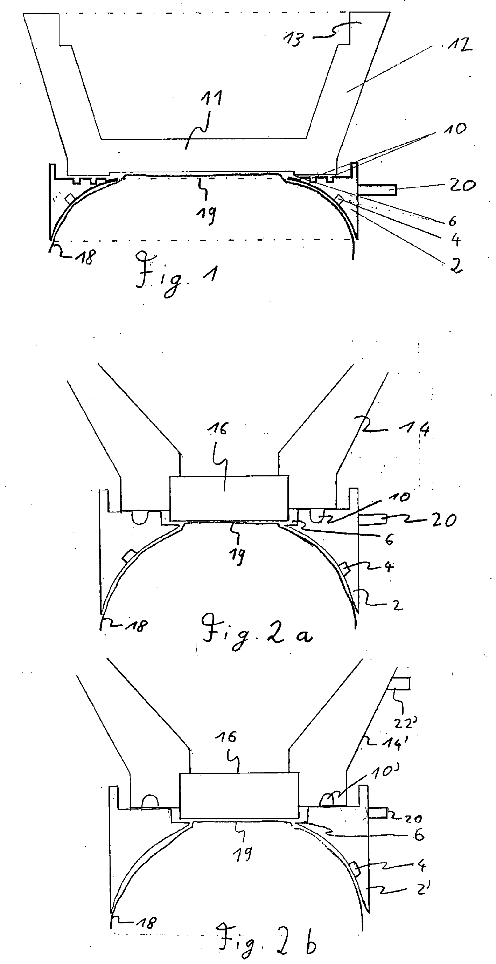

Figur 1 zeigt eine Augensaugeinrichtung (d.h. Saugring) 2 mit einem ersten Saugbereich 4, der dazu ausgebildet ist, im Einsatz die Augensaugeinrichtung 2 an ein Auge 18 zu saugen, und einem zweiten Saugbereich 10, der dazu ausgebildet ist, im Einsatz ein Funktionselement 12 anzusaugen. Das Funktionselement 12 umfasst ein optisches Element 11, das als Linse oder Platte 11 ausgebildet ist. Ferner umfasst das Funktionselement 12 einen Anschlussbereich 13, mit dem das Funktionselement an eine optische Einrichtung (nicht gezeigt) eines Lasersystems, beispielsweise eines Femtosekunden-Lasersystems, gekoppelt werden kann.

Ferner umfasst die Augensaugeinrichtung einen dritten Saugbereich, der mit dem Raum zwischen dem Funktionselement 12 und der Hornhaut 19 eines Auges 18 in Fluidverbindung steht und diesen zumindest teilweise evakuiert, so dass zumindest ein Teil der Hornhaut an dem Funktionselement 12 anliegt. Der Bereich, an dem die Hornhaut 19 anliegt, kann als Applanationslinse bzw. Applanationsplatte 11 ausgebildet sein. Die Augensaugeinrichtung umfasst eine Mehrzahl von Unterdruckzuführungen 20, 22, 24 (in Figur 1 ist nur eine erkennbar), die an einer oder je an einer Saugpumpe oder an einer Saugpumpe mit drei separaten Regelungseinrichtungen bzw. drei separaten Regelungsventilen angeschlossen sind. Dadurch kann in dem ersten Saugbereich 4, dem zweiten Saugbereich 10 und dem dritten Saugbereich 6 ein unterschiedlicher Unterdruck erzeugt werden. Im zweiten Saugbereich 10 kann ein hoher Unterdruck eingestellt werden, damit das Funktionselement 12 fest an der Augensaugeinrichtung angeordnet ist. Im ersten Saugbereich 4 und/oder im dritten Saugbereich 6 kann ein niedrigerer Unterdruck eingestellt werden, damit das Auge 18 nicht verletzt wird. Der Operateur kann ferner vor der Operation lediglich einen Unterdruck im zweiten Saugbereich 10 anlegen, um das Funktionselement 12 an der Augensaugeinrichtung 2 anzuordnen. Beim Positionieren der Augensaugeinrichtung 2 an dem Auge 18 des Patienten kann im ersten Saugbereich 4 ein Unterdruck erzeugt werden, um die Augensaugeinrichtung 2 am Auge 18 sicher anzuordnen. Schließlich kann ein Unterdruck im dritten Saugbereich 6 erzeugt werden, damit die Hornhaut 19 des Auges 18 sicher am Funktionselement 12 angeordnet ist. Es ist jedoch auch denkbar, zuerst die Augensaugeinrichtung 2 am Auge 18 anzuordnen und anschließend ein Vakuum im zweiten Saugbereich 10 zu erzeugen, um das Funktionselement 12 anzuordnen.

Die Vorrichtung nach Fig. 1 vermeidet eine Verletzung des Auges, da im ersten Saugbereich 4 ein anderer Unterdruck eingestellt werden kann als im zweiten Saugbereich 10 und/oder im dritten Saugbereich 6. Ferner wird eine Redundanz geschaffen, da die Augensaugeinrichtung sowohl durch den ersten Saugbereich 4 als auch durch den dritten Saugbereich 6 am Auge gehalten wird.

Der erste Saugbereich 4 ist als umlaufende Nut in der Augensaugeinrichtung ausgebildet. Es sind jedoch auch andere Ausgestaltungen möglich, beispielsweise eine Mehrzahl von umlaufenden Nuten, eine oder mehrere umlaufende oder nicht umlaufende Einbuchtungen oder eine Mehrzahl von Öffnungen. Der zweite Saugbereich 10 ist durch zwei kreisförmig umlaufende Nuten an der Vorderseite oder Oberseite der Augensaugeinrichtung ausgebildet. Auch hier sind die zuvor bezüglich des ersten Saugbereiches beschriebenen Ausgestaltungen möglich. Der dritte Saugbereich 6 befindet sich bei dem ersten Beispiel im Übergangsbereich von der Augensaugeinrichtung 2 zum Funktionselement 12. Dadurch kann sichergestellt werden, dass ein Teil der Hornhaut 19 an die Oberfläche des Funktionselementes 12 gesaugt wird.

Das Funktionselement 12 lässt sich einfach wechseln, ohne dass spezielle Werkzeuge und/oder aufwändige Bedienschritte dafür erforderlich sind. Das Funktionselement 12 kann im Einsatz einfach durch ein anderes ausgetauscht werden, das beispielsweise ein anderes optisches Element 11, Applanationselement 11 und/oder einen anderen Anschlussbereich 13 umfasst.

Figur 2a zeigt ein zweites Beispiel einer augenchirurgischen Vorrichtung, wobei gleiche Bezugszeichen gleiche Elemente bezeichnen. Bezüglich der Ausgestaltung des ersten, zweiten und dritten Saugbereiches sowie der Unterdruckzuführungen wird auf die Beschreibung des Beispiels gemäß Figur 1 verwiesen.

Im Gegensatz zum Beispiel nach Figur 1 wird bei dem Beispiel nach Figur 2a ein Halterungselement 14 durch einen Unterdruck im zweiten Saugbereich 10 an der Augensaugeinrichtung 2 angeordnet. Das Halterungselement 14 kann ein beliebiges Element sein, an dem weitere Elemente angeordnet oder befestigt werden können. Das Halterungselement 14 umfasst an seinem der Augensaugeinrichtung 2 zugewandten Bereich eine kreisförmig umlaufende Ausnehmung, in der ein optisches Element 16 angeordnet ist. Das optische Element 16 kann als Applanationselement oder als Applanationslinse ausgebildet sein. Das Halterungselement 14 umfasst auch einen (nicht gezeigten) Anschlussbereich, mit dem das Halterungselement mit der Optik eines Lasersystems gekoppelt werden kann.

Durch den Unterdruck im dritten Saugbereich 6 wird die Hornhaut 19 an das am Halterungselement 14 angeordnete optische Element 16 gesaugt.

Figur 2b zeigt ein drittes Beispiel einer augenchirurgischen Vorrichtung, die dem zweiten Beispiel ähnelt. Im Gegensatz zum zweiten Beispiel umfasst die Augensaugeinrichtung 2' bei dem dritten Beispiel keinen zweiten Saugbereich. Der zweite Saugbereich 10' ist in dem Funktionselement 14' angeordnet. Über die zweite Unterdruckzuführung 22' wird ein Unterdruck in dem zweiten Saugbereich 10' erzeugt. Die übrigen Ausgestaltungen der Augensaugeinrichtung 2' und des Funktionselementes 14' entsprechen den Ausgestaltungen der Augensaugeinrichtung 2 und des Funktionselementes 14 des zweiten Beispiels.

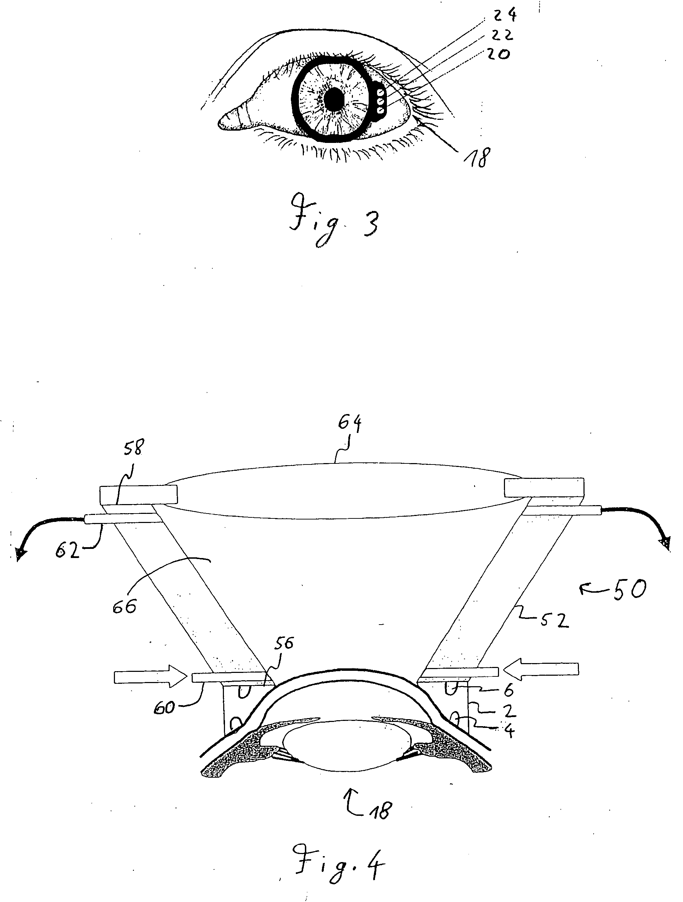

Figur 3 zeigt eine Frontansicht der beispielhaften Vorrichtung ohne aufgesetztes Funktionselement im Einsatz.

Die Augensaugeinrichtung umfasst eine erste Unterdruckzuführung 20, die mit dem ersten Saugbereich verbunden ist, eine zweite Unterdruckzuführung 22, die mit dem zweiten Saugbereich verbunden ist, und eine dritte Unterdruckzuführung 24, die mit dem dritten Saugbereich verbunden ist. Die Augensaugeinrichtung muss nicht im Wesentlichen kreisrund sein. Sie kann beispielsweise auch als elliptischer Ring oder Polygonring ausgebildet sein.

Figur 4 zeigt eine Ausführungsform eines erfindungsgemäßen Saugringes 2, bei dem sich während der Behandlung des Auges 18 ein Fluid zwischen einer Fokussierlinse 64 und der Hornhaut des Auges 18 befindet. Die Augensaugeinrichtung 2 wird mithilfe eines ersten Unterdruckbereichs 4 an das Auge 18 gekoppelt, beispielsweise an die Sklera oder den Limbus. Der Bereich der Augensaugeinrichtung 2, der das Auge 18 berührt, ist der anatomischen Form des Auges 18 in diesem Bereich nachgebildet, damit der Augendruck nicht oder nur geringfügig erhöht wird. Die Augensaugeinrichtung 2 und die daran angeordneten Elemente berühren und/oder bedecken das Auge 18 jedoch nicht in dem zu behandelnden Bereich der Hornhaut, wodurch sich eine numerische Apertur von etwa 10 mm ergibt.

Die Augensaugeinrichtung 2 umfasst einen ersten Saugbereich 4, der ein Gefäßelement 50 an dessen ersten axialen Ende 56 ansaugt. Dem ersten axialen Ende 56 des Gefäßelementes 50 entgegengesetzt befindet sich ein zweites axiales Ende 58, an dem eine Fokussierlinse 64 angeordnet sein kann. Eine Wand 52 erstreckt sich von dem ersten axialen Ende 56 zum zweiten axialen Ende 58 des Gefäßelementes 50 und um die Längsachse des Gefäßelementes 50 in axialer Richtung. Das Gefäßelement ist somit seitlich durch die Wand 52, nach oben durch die Fokussierlinse 64 und nach unten durch die Hornhaut des Auges 18 geschlossen.

Die Wand 52 weist eine erste Öffnung 60, durch die ein Fluid zugeführt werden kann, und eine zweite Öffnung 62 auf, durch die ein Fluid abgeführt werden kann. Wird durch die erste Öffnung 60 ein Fluid zugeführt, entweicht die Luft im Inneren 66 des Gefäßelementes durch die zweite Öffnung 62. Das dem Inneren 66 des Gefäßelementes zugeführte Fluid weist vorzugsweise etwa einen ähnlichen oder den gleichen Brechungsindex $\eta$ wie die Cornea auf, deren Brechungsindex $\eta_{cornea}$ etwa 1,376 beträgt. Es ist aber auch möglich, Wasser zu verwenden, dessen Brechungsindex $\eta_{wasser}$ etwa 1,333 beträgt. Durch die Anpassung der Brechungskoeffizienten entsteht keine optische Aberration des Lichtes beim Übergang in die Hornhaut, wodurch eine gute Fokussierbarkeit des Laserstrahles und eine hohe optische Qualität des Laserstrahles gewährleistet ist.

Befindet sich in der Umgebung des ersten Saugbereiches 6 ein Feuchtigkeitsfilm, wird, wie zuvor erwähnt, eine besonders gute Abdichtung des ersten Saugbereiches 6 gewährleistet, wodurch die Augensaugeinrichtung besonders sicher an das Auge gekoppelt wird. Je höher die Viskosität der Flüssigkeit ist, desto besser die zusätzliche Dichtwirkung.

Ferner ist bei dieser Ausführungsform, wie zuvor erwähnt, kein Applanationselement erforderlich, was dazu führt, dass der Augendruck während der Behandlung nicht erhöht wird. Ferner kann keine Aberration, beispielsweise ein Wellenfrontfehler, aufgrund einer sphärischen oder asphärischen Applanation wie beim Einsatz eines Applanationselementes entstehen. Folglich wird eine Laserstrahlung mit einem niedrigen Wellenfrontfehler erreicht, die hinsichtlich der Fokussierung vorteilhaft ist, da die Fokuspositionen weniger stark streuen und die Laserstrahlung auf einen kleineren Fokusbereich konzentriert wird.

Das Gefäßelement 50 und die Augensaugeinrichtung 2 können integral ausgebildet sein oder form- oder reibschlüssig miteinander verbunden werden.

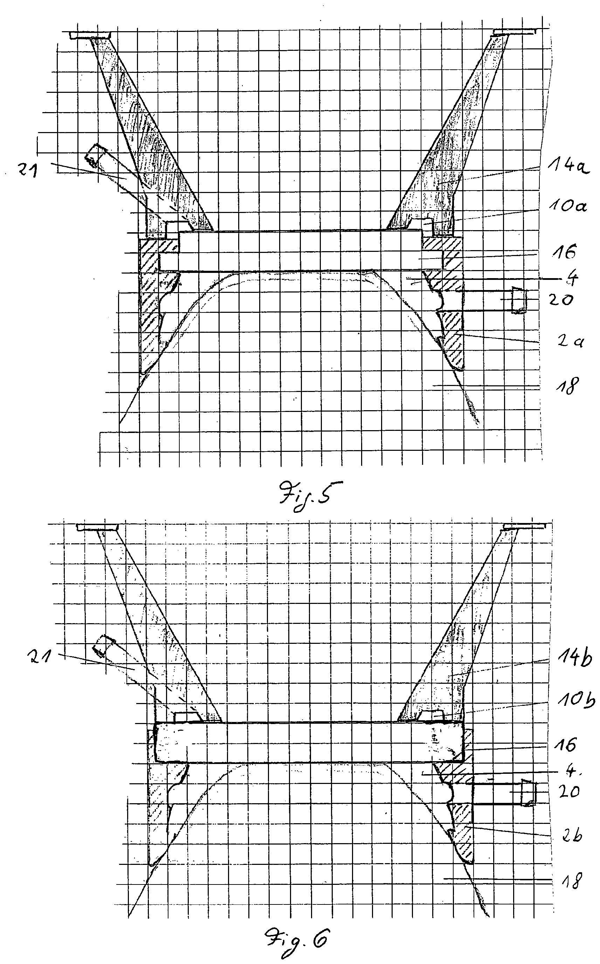

Figuren 5 und 6 zeigen weitere Beispiele einer augenchirurgischen Vorrichtung. Das optische Element 16 ist ein Applanationselement. Das Applanationselement 16 ist an der Augensaugeinrichtung 2a, 2b befestigt. Wie zuvor erwähnt wurde, muss das Applanationselement 16 nach einer Operation, nachdem es der Laserstrahlung ausgesetzt war, getauscht werden. Die Augensaugeinrichtung 2a, 2b kann mit dem daran befestigten Applanationselement 16 als steriler Einwegartikel bereitgestellt werden. Dadurch lässt sich eine kostengünstigere Augensaugeinrichtung erzielen. Ferner kann bei einer derartigen Augensaugeinrichtung die mechanische Toleranzkette auf das Applanationselement 16 und das angesaugte, wiederverwendbare Halterungselement 14a, 14b reduziert werden, das auch als Adaptionskegel bezeichnet wird. Durch die reduzierte Toleranzkette wird das Behandlungsergebnis verbessert.

Durch die erste Unterdruckzuführung 20 wird, wie zuvor beschrieben, ein Unterdruck in dem ersten Saugbereich 4 erzeugt, so dass die Augensaugeinrichtung 2a, 2b am Auge 18 gehalten wird. Eine zweite Unterdruckzuführung 21 erzeugt einen zweiten Unterdruck 10a, 10b in dem zweiten Saugbereich 10a, 10b, so dass das Halterungselement bzw. der Adaptionskegel 14a, 14b sicher an dem Applanationselement 16 gehalten wird.

Bei dem Beispiel gemäß Figur 5 berührt das Halterungselement bzw. der Adaptionskegel 14a sowohl das Applanationselement 16 als auch die Augensaugeinrichtung 2a. Bei dieser Ausführungsform der Augensaugeinrichtung 2a wird der Rand des Applanationselementes 16 vollständig von der Augensaugeinrichtung umschlossen.

Bei dem Beispiel gemäß Figur 6 berührt das Halterungselement bzw. der Adaptionskegel 14b lediglich das Applanationselement 16 und nicht die Augensaugeinrichtung 2b. Bei dieser Ausführungsform der Augensaugeinrichtung 2b kann das Applanationselement 16 nur mit seinem unteren Rand auf der Augensaugeinrichtung 2b aufliegen. Es kann zusätzlich, beispielsweise mittels eines Klebstoffes, befestigt werden. Auch bei dieser Ausführungsform ist es möglich, dass die Augensaugeinrichtung 2b das Applanationselement 16 vollständig umschließt, so dass kein Klebstoff erforderlich ist. Das Halterungselement 14b muss in diesem Fall einen an seiner dem Applanationselement 16 zugewandten Seite kleineren Durchmesser als das Applanationselement aufweisen.

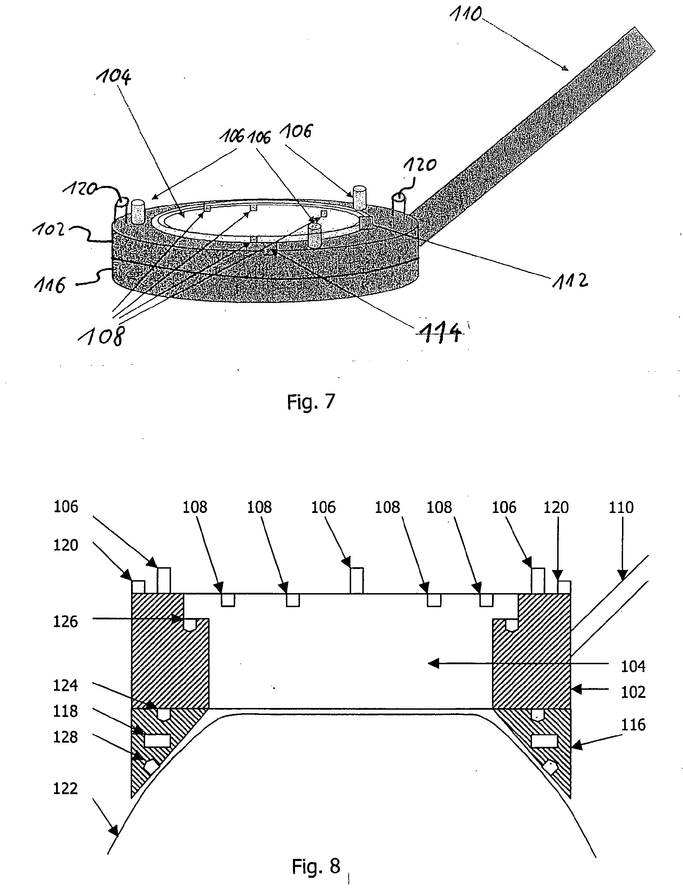

Figuren 7 und 8 zeigen ein weiteres Beispiel einer augenchirurgischen Vorrichtung, die eine Augensaugeinrichtung 116, ein Halterungselement 102, ein Applanationselement 104, eine Zuführeinrichtung 110 mit zumindest einer Unterdruckleitung sowie einer Stromversorgung und einer Signalverbindung für zumindest ein Messmittel umfasst. An der Unterseite der Augensaugeinrichtung 116 befindet sich ein erster Saugbereich 128, mit dem die Augensaugeinrichtung 116 gegenüber dem Auge 122 fixiert wird. Die Augensaugeinrichtung 116 kann über einen zweiten Saugbereich 124, in dem ein Unterdruck herrscht, das Halterungselement 102 fixieren. Das Halterungselement 102 fixiert über einen dritten, optionalen Saugbereich 126 das Applanationselement 104. Durch das Applanationselement 104 wird die Strahlung eines (nicht gezeigten) Lasersystems, beispielsweise eines Femtosekunden-Lasersystems, eingekoppelt. Das Applanationselement kann auch als Linse ausgebildet sein. Das Applanationselement 104 liegt an seinem unteren Ende an der Hornhaut des Auges 122 an, wodurch die Position der Hornhaut festgelegt wird.

An der Oberseite des Halterungselementes 102 ist eine Mehrzahl von mechanischen Führungen 106 zum Ankoppeln des Strahlenganges des Lasers und/oder eines Verschlussmechanismus ausgebildet. Die mechanischen Führungen 106 können einen Kraftsensor (nicht dargestellt) umfassen. Ferner kann der Kraftsensor an den mechanischen Führungen angeordnet werden, wobei die Optik des Lasers in diesem Fall an die Kraftsensoren gekoppelt wird. Der Kraftsensor kann beispielsweise ein piezoelektrischer Sensor sein oder er kann mittels Dehnungsmessstreifen aufgebaut sein.

An dem Applanationselement 104 können Messmittel zum Messen des Augeninnendruckes angeordnet sein. Die Messmittel können durch mikroelektromechanische Systeme (MEM-Systeme) bereitgestellt werden. Am Rand des Applanationselementes 104 befindet sich eine Mehrzahl von Fasersensoren 108 zum Durchführen eines Spektroskopieverfahrens, beispielsweise im nahen Infrarotbereich, und/oder zum Durchführen eines Verfahrens zur Bestimmung der Lichtstreuung. Mit diesen Fasersensoren können Eigenschaften der Hornhaut, beispielsweise deren Wassergehalt, bestimmt werden. Durch die Bestimmung der Lichtstreuung kann auch die Transparenz der Hornhaut bestimmt werden. Bei der fünften Ausführungsform ist jedoch ein separater Transparenzsensor 114 vorgesehen, der beispielsweise durch Bestimmung der Lichtstreuung der Hornhaut deren Transparenz ermittelt. Eine mechanische Spektroskopieeinrichtung 112 ermittelt, beispielsweise aufgrund mechanischer Resonanz, die biomechanischen Eigenschaften der Hornhaut. Über die Zuführeinrichtung 110 wird der Augensaugeinrichtung Unterdruck zugeführt. Ferner wird mit der Zuführeinrichtung 110 eine Stromversorgung gewährleistet, und die Messsignale der Messmittel werden an eine (nicht gezeigte) Auswerteeinrichtung über ein elektrisches Kabel, eine Glasfaser und/oder Funk übertragen. An dem Applanationselement 104 und der Augensaugeinrichtung 102 können Kontaktelemente vorgesehen werden, um elektrische Signale zu übertagen und eine Stromversorgung bereitzustellen.

An dem Halterungselement 102 sind ferner Positionsmessmittel 120 vorgesehen, um die Position der Augensaugeinrichtung 116 bzw. des Halterungselementes 102 im Raum zu bestimmen. Die Positionsmessmittel 120 können beispielsweise akustische oder optische Sensoren sein, die die Position gegenüber einer Referenzgeometrie bestimmen. Ferner kann es sich bei den Positionssensoren 120 um rein passive Sensoren handeln, die einen von einem Referenzort ausgestrahlten Strahl empfangen, wobei auf Basis des empfangenen Signals die Position der Augensaugeinrichtung 116 bzw. des Halterungselementes 102 bestimmt wird. Ebenso können die Positionsmessmittel 120 rein aktive Elemente sein, die einen optischen oder akustischen Strahl aussenden, der von einer entsprechenden Empfangseinrichtung an einem Referenzort empfangen wird, wobei auf Basis des empfangenen Signals die Position der Positionsmessmittel 120 im Raum bestimmt wird. An der Augensaugeinrichtung 102 können auch Referenzmarken angeordnet sein, die von einer im Raum angeordneten Kamera erfasst werden. Ebenso können im Inneren der Augensaugeinrichtung optische Sensoren angeordnet sein, die die Position des Auges erfassen. Mit Hilfe der erfassten Position der Augen gegenüber dem Augensaugring 102 und der über die Positionsmessmittel 120 erfassten Position des Augensaugringes im Raum kann die Position des Auges 122 im Raum bestimmt werden.

Die Mittel zum Bestimmen der Position des Saugringes im Raum können mit einer (nicht gezeigten) Positionssteuerung einer Behandlungsliege oder einer (nicht gezeigten) Positionssteuerung eines Lasersystems gekoppelt werden.

Die Augensaugeinrichtung 116 berührt im Einsatz das Auge. In der Augensaugeinrichtung 116 ist ein Kraftsensor 118 integriert, um die auf das Auge wirkende Kraft zu messen, woraus der Augeninnendruck bestimmt werden kann. In der Augensaugeinrichtung 116 kann eine Mehrzahl von Kraftsensoren 118 angeordnet sein. Dadurch kann ein Kraftprofil entlang dem Umfang des Kontaktelementes 116 erstellt werden, wodurch das Hornhautprofil ermittelt werden kann und demgemäss der Augeninnendruck genauer ermittelt werden kann.

Die Augensaugeinrichtung 116 oder das Halterungselement 102 kann ferner einen vierten Saugbereich (nicht gezeigt) umfassen, der den Bereich zwischen dem Applanationselement und der Hornhaut evakuiert, damit die Hornhaut des Auges 122 sicher an dem Applanationselement 104 anliegt.

Die Augensaugeinrichtung 116 und das Halterungselement 102 können integral ausgebildet sein oder kraft- oder formschlüssig verbunden sein.

## Patentansprüche

1. Vorrichtung für die Augenchirurgie, wobei die Vorrichtung dazu ausgebildet ist, während einer Behandlung eines Auges ein von Luft verschiedenes Fluid zwischen einer Fokussierlinse (64) und der Hornhaut des Auges bereitzuhalten, wobei die Vorrichtung umfasst:

  - einen auf ein zu behandelndes Auge (18) aufsetzbaren Saugring (2) mit einem ersten Saugbereich (4), der dazu ausgebildet ist, durch Unterdruckerzeugung in dem ersten Saugbereich den Saugring an das Auge (18) zu saugen, und
  - ein integral mit dem Saugring ausgebildetes oder mittels Unterdruck oder mechanisch an den Saugring ankoppelbares Gefäßelement (50) mit einer Längsachse und einem Innenraum (66) zur Aufnahme des Fluids, wobei das Gefäßelement eine sich von einem ersten axialen Ende (56) zu einen entgegengesetzten zweiten axialen Ende (58) des Gefäßelements erstreckende, um die Längsachse verlaufende Wand (52) aufweist, durch welche es seitlich geschlossen ist, und wobei in der Wand eine erste Öffnung (60) zur Zuführung des Fluids in den Innenraum und eine zweite Öffnung (82) zur Entlüftung des Innenraums vorgesehen sind, wobei die beiden Öffnungen axial zueinander versetzt in der Wand angeordnet sind.

## Claims

1. Apparatus for eye surgery, wherein the apparatus is embodied to keep available, during a treatment of an eye, a fluid, which differs from air, between a focusing lens (64) and the cornea of the eye, wherein the apparatus comprises:

  - a suction ring (2), which can be placed onto an eye (18) to be treated, comprising a first suction region (4) that is embodied to suck the suction ring onto the eye (18) by producing negative pressure in the first suction region and
  - a vessel element (50) that is integrally embodied with the suction ring or coupleable to the suction ring by means of negative pressure or mechanical means, said vessel element having a longitudinal axis and an interior (66) for receiving the fluid, wherein the vessel element comprises a wall (52) extending about the longitudinal axis from a first axial end (56) to an opposite second axial end (58) of the vessel element, said wall laterally closing off said vessel element, and wherein a first aperture (60) for supplying the fluid into the interior and a second aperture (82) for venting the interior are provided in the wall, wherein the two apertures are arranged axially offset from one another in the wall.

## Revendications

1. Arrangement pour la chirurgie ophtalmologique, l'arrangement étant configuré pour, pendant un traitement d'un oeil, tenir prêt un fluide différent de l'air entre une ligne de focalisation (64) et la cornée de l'oeil, l'arrangement comprenant :

  - une bague d'aspiration (2) pouvant être posée sur l'oeil (18) à traiter, pourvue d'une première zone d'aspiration (4) qui est configurée pour aspirer la bague d'aspiration contre l'oeil (18) en générant une dépression dans la première zone d'aspiration et
  - un élément formant récipient (50), réalisé d'un seul tenant avec la bague d'aspiration ou pouvant être accouplé par dépression ou mécaniquement à la bague d'aspiration, comprenant un axe longitudinal et un espace intérieur (66) destiné à accueillir le fluide, l'élément formant récipient possédant une paroi (52) qui s'étend depuis une première extrémité axiale (56) jusqu'à une deuxième extrémité axiale (58) à l'opposé de la première extrémité axiale en suivant l'axe longitudinal, par laquelle il est fermé latéralement, et une première ouverture (60) servant à l'acheminement du fluide dans l'espace intérieur et une deuxième ouverture (82) servant à la purge d'air de l'espace intérieur se trouvant sur la paroi, les deux ouvertures étant disposées dans la paroi en étant décalées axialement l'une de l'autre.

Fig. 1

Fig. 2 a

Fig. 2 b

Fig. 3

Fig. 4

Fig. 5

Fig. 6

Fig. 7

Fig. 8

**EP 2 133 048 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5336215 A
- US 5549632 A
- EP 0993814 A1
- US 6342053 B1
- US 6344040 B1
- WO 03002008 A1
- WO 0041660 A1
- WO 03001991 A1
- WO 2006121066 A1